(19) Europäisches Patentamt
European Patent Office
Office européen des brevets

(11) Veröffentlichungsnummer : **0 051 741**
**B1**

(12) # EUROPÄISCHE PATENTSCHRIFT

(45) Veröffentlichungstag der Patentschrift : **13.06.84**

(51) Int. Cl.³ : **C 01 B 33/28**, B 01 J 29/28, C 07 C 2/66, C 07 C 1/20

(21) Anmeldenummer : **81107901.1**

(22) Anmeldetag : **05.10.81**

(54) **Verfahren zur Herstellung eines kristallinen Aluminiumsilikates (Zeoliths) und dessen Verwendung als Katalysator.**

(30) Priorität : **06.11.80 DE 3041847**

(43) Veröffentlichungstag der Anmeldung : **19.05.82 Patentblatt 82/20**

(45) Bekanntmachung des Hinweises auf die Patenterteilung : **13.06.84 Patentblatt 84/24**

(84) Benannte Vertragsstaaten : **BE DE FR GB IT NL**

(56) Entgegenhaltungen :
**FR-A- 2 325 603**
**GB-A- 1 567 948**
**GB-A- 2 018 232**
**US-A- 4 175 114**
**CHEMICAL ABSTRACTS, Band 93, Nr. 6, 11. August 1980, Zusammenfassung 58810a, Seite 814, rechte Spalte COLUMBUS, OHIO (US)**
**ZEITSCHRIFT FÜR CHEMIE, 13. Jahrgang. Heft 3, 1973 LEIPZIG (DD) F. WOLF et al.: "Über den Einfluss organischer Verbindungen auf die hydrothermale Synthese zeolithischer Molekularsiebe der Typen A,X ud Y"**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Hoelderich, Wolfgang, Dr.**
**Mannheimer Strasse 18 C**
**D-6710 Frankenthal (DE)**
Erfinder : **Marosi, Laszlo, Dr.**
**Leuschnerstrasse 32**
**D-6700 Ludwigshafen (DE)**
Erfinder : **Mross, Wolf Dieter, Dr.**
**Anselm-Feuerbach-Strasse 21**
**D-6710 Frankenthal (DE)**
Erfinder : **Schwarzmann, Matthias, Dr.**
**Carl-Bosch-Strasse 54**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 741 B1

Jouve, 18, rue St-Denis, 75001 Paris, France

**Beschreibung**

Die Erfindung betrifft ein Verfahren zur Herstellung eines kristallinen Aluminosilikates vom Pentasiltyp.

Kristalline Aluminosilikate, wie Zeolithe, natürlicher als auch synthetischer Herkunft, haben sich als katalytisch wirksame Stoffe für verschiedene Arten von Kohlenwasserstoffumwandlung, z. B. beim Cracken von Kohlenwasserstoffen, erwiesen. Aber auch als Ionenaustauscher und Molekularsiebe finden Zeolithe technische Verwendung.

Kristalline Aluminosilikate, wie Zeolithe besitzen eine hochgeordnete Struktur mit einem starren dreidimensionalen Netzwerk von $SiO_4$- und $AlO_4$-Tetraedern, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall z. B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt.

In den letzten Jahren gewannen kristalline Aluminosilikate mit einem hohen $SiO_2/Al_2O_3$-Verhältnis $\geqslant 11$ zunehmendes Interesse. Derartige Zeolithe zeichnen sich durch hohe thermische Stabilität und außerordentlich hohe Acidität aus. Die Synthese von Zeolithen mit Pentasil-Struktur erfolgt in bekannter Weise aus einer Silicium- und einer Aluminiumkomponente in Gegenwart von voluminösen quaternären organischen Ammoniumverbindungen und Alkaliverbindungen als Mineralisierungsmittel. Ein derartiges Verfahren ist z. B. in US-A-3 702 886 beschrieben. Die großen Ammoniumionen sollen als Muster für die gewünschte Zeolithstruktur wirken und ermöglichen die Synthese von kristallinen Aluminiumsilikaten mit $SiO_2/Al_2O_3$-Verhältnissen von über 100, z. B. bis zu 3 000. In Gegenwart von derartigen Zeolithen kann man z. B. die Umwandlung von Methanol unter C.Verknüpfung in Olefine ausführen (US-A-3 911 041) oder die Alkylierung des Benzols mit Ethylen zu Ethylbensol katalysieren (US-A-3 751 504).

Die Verwendung der quaternären Amine als Lösungsmittel ist technisch problematisch. Die Geruchsbelästigung und ätzende Wirkung der Amine erfordern aufwendige Sicherheitsmaßnahmen. Ein Teil des Amins wird während der Synthese zersetzt und geht für weitere Synthesen verloren.

Es wurde nun gefunden, daß man kristalline Aluminosilikate vom Pentasiltyp (Zeolithe) in einfacherer Weise und ohne die obenbeschriebenen Nachteile durch hydrothermale Kristallisation von $SiO_2$ und $Al(OH)_3$ in Gegenwart von Alkalisalzen bei Temperaturen von 100 bis 250 °C herstellen kann, wenn man die Kristallisation in etherischer oder wäßrig etherischer Mischung ausführt.

Eine bevorzugte Ausführungsform zur Synthese dieser aminfreien kristallinen Aluminosilikate (Zeolithe) besteht darin, daß man eine Reaktionsmischung aus $SiO_2$, z. B. eine käufliche pyrogene Kieselsäure, frisch gefälltem $Al(OH)_3$ und NaOH in einer Ether/Wasser-Mischung von 50 : 50 Gew.% 3 bis 5 Tage bei Temperaturen zwischen 150 und 170 °C unter autogenem Druck in einem Rührautoklaven umsetzt. Das zunächst erhaltene Reaktionsprodukt kann vor der Calcinierung noch Ether enthalten, anstelle der in den intrakristallinen Poren enthaltenden $H_2O$-Molekel. Alle Reste organischer Verbindungen werden durch Calcinieren entfernt, so daß nur noch die freien Alkaliionen im kristallinen Aluminosilikat enthalten sind.

Das Molverhältnis von $SiO_2$ zu $Al_2O_3$ in der Reaktionsmischung wird zwischen 20 und 200 eingestellt, bevorzugt bei 40-75.

Der Anteil an Alkalihydroxid liegt bei 1 bis 3 Mol pro Mol Äquivalent $Al_2O_3$, bevorzugt bei 1,5 Mol. Es können auch Impfkristalle zur Reaktionsführung und -beschleunigung zugesetzt werden.

Als Lösungsmittel kann man allgemein Ether, sowohl lineare als auch cyclische Ether mit einer ($-CH_2-CH_2-O$)-Gruppierung wie Mono-, Di,- Tri- und Tetraethylenglykoldimethylether (Glyme), Diethylether, Tetrahydrofuran, Dioxan oder ein Gemisch von Tri- bis Dekaethylenglykol-methylisopropylethern des mittleren Ethoxylierungsgrades 5,5 der Formel $CH_3O-(CH_2-CH_2-O)_{5,5}-C_3H_7$, Diethylenglykol-methyl-isopropylether oder ein Gemisch von Polyethylenglykol-methyl-isopropylethern des mittleren Ethoxylierungsgrades 21 oder ein Gemisch von Tri- bis Dekaethylenglykoldimethylethern des mittleren Ethoxylierungsgrades 5,5 der Formel $CH_3O(-CH_2-CH_2-O)_{5,5}$ oder ein Gemisch von ethoxylierten Oxoalkoholen mit mittlerem Molekulargewicht von 200, 600, 6 000 sowie lineare mit einer ($CH_2-CH-CH_2-O$)-Gruppierung sowie lineare mit einer ($-CH_2-O$)-Gruppierung wie Dimethoxymethan. Bei Verwendung von Ethergemischen wie Sepasolv®, Selexol® und den Plurioltypen®, ist die Herstellung schwieriger als bei Verwendung von Ethern mit definierter Kettenlänge. Bei den Glymen (Mono- bis Tetra-) und Tetrahydrofuran als Lösungsmittel erhält man reine kristalline Aluminosilikate mit gut geformten Kristallen, deren Habitus sich je nach Wahl des Ethers ändert. Die Ausbildung gutgeformter Kristalle von der Größe 2/u-15/u ist ein Vorteil dieser Herstellungsweise in Ethern.

Die molare Zusammensetzung der Reaktionsmischung wird zweckmäßig wie folgt eingestellt:

$SiO_2/Al_2O_3$ = 20-200, vorzugsweise 40-75
$M_2O/Al_2O_3$ = > 1, vorzugsweise 1,5-2 und
$ROR/Al_2O_3$ = 18-390,

® eingetragene Warenzeichen.

wobei M = Alkali, insbesondere Na und ROR einen Ether bedeutet. Die Konzentration der Ether in $H_2O$ kann zwischen 10 und 100 Gew.% liegen, bevorzugt 50 Gew.%.

Das erfindungsgemäße Verfahren wird im allgemeinen bei Reaktionstemperaturen im Bereich von 100 bis 250 °C, vorzugsweise zwischen 150 und 190 °C, durchgeführt. Die Reaktionsdauer hängt unter anderem von der Temperatur und vom Zusatz von Impfkristallen ab. Sie kann z. B. bei Temperaturen zwischen 150 und 190 °C zwischen 1 und 5 Tagen betragen. Die Reaktion wird vorteilhafterweise unter dem Eigendruck in einem Rührautoklaven durchgeführt.

Nach der Reaktion wird das Reaktionsprodukt zweckmäßigerweise abfiltriert, mit Wasser gut ausgewaschen und bei 110 °C 16 h getrocknet. Anschließend wird das Produkt bei 550 °C/20 h calciniert, um den noch eingeschlossenen Ether herauszubrennen und den Zeolith zu dehydratisieren. Die Überführung der Na-Form in die katalytisch aktive H-Form kann nach bekannten Austauschverfahren z. B. mit Ammoniumsalzen erfolgen.

Die allgemeine Formel des kristallinen Aluminosilikates (Zeolithen) lautet :

$$0{,}01\text{-}1{,}3\ Na_2O : Al_2O_3 : \geq 15\ SiO_2 : 0\text{-}45\ H_2O.$$

Die erfindungsgemäß in Ether hergestellten Zeolithe zeigen ein Röntgenbeugungsdiagramm, das sie als Mitglieder der Pentasilfamilie ausweist. Die abgetrennte etherische Mutterlauge kann für weitere Synthesen verwendet werden. Die nach Austausch in H-Form erhaltenen. Zeolithe können mit Matrixmaterialien z. B. Pural SB® bis zum Verhältnis 90 : 10 verstrangt und als Katalysatoren für Kohlenwasserstoffumwandlung eingesetzt weden, z. B. bei der Gasphasenalkylierung des Benzols mit Ethylen zu Ethylbenzol bei Temperaturen zwischen 300 und 450 °C, WHSV = 1-10 $h^{-1}$ (bezogen auf $C_6H_6$) und $C_6H_6 : C_2H_4$ = 1-7 oder bei der Umwandlung von Alkoholen und Ethern, insbesondere Methanol und/oder Dimethylether zu Olefinen bei Temperaturen zwischen 350-450 °C und WHSV = 0,5-10 $h^{-1}$ (bezogen auf $CH_3OH$).

Die in den Beispielen angegebenen Analysenwerte beziehen sich auf Trockenbasis. Die Substanzen wurden vor der chemischen Analyse bei 550 °C/20 h calciniert. Die Differenz zu 100 % ergibt sich durch adsorbiertes Wasser.

## Beispiele 1-10

In 1 000 g Ether/$H_2O$-Gemisch (50 : 50) werden bei 60 °C 80 g Aerosil® eingeführt. Aus 25 g $Al(NO_3)_3$ 9 $H_2O$ läßt sich mit $NH_3Al(OH)_3$ fällen. Dieses frisch gefällte $Al(OH)_3$ wird abfiltriert, gut mit Wasser ausgewaschen und in 200 g Ether/$H_2O$-Gemisch (50 : 50) suspendiert. Diese Suspension wird in obige suspension eingetragen. Hinzu werden 3,4-5,3 g NaOH in 20 g $H_2O$ gelöst gegeben. Dabei tritt meist eine Verdickung des Reaktionsgemisches ein. Die homogen gerührte Mischung wird in einem Rührautoklaven 5 Tage bei 150-170 °C unter autogenem Druck umgesetzt. Nach Abkühlen des Reaktionsgemisches wird das kristalline Produkt abfiltriert mit 10 l $H_2O$ ausgewaschen, 16 h bei 110 °C getrocknet und 20 h bei 500 °C calciniert.

Beispiele 1-10 sind in Tabelle 1 wiedergegeben.

## Beispiele 11-13

Die Beispiele 11-13 zeigen die Herstellung von Zeolithen in Diglym (Diethylglykoldimethylether) mit variablem $SiO_2/Al_2O_3$-Verhältnis.

® eingetragene Warenzeichen.

(Siehe Tabellen 1, 2, Seite 4 ff.)

Tabelle 1

| Beispiel | Ether | eingesetztes $SiO_2/Al_2O_3$-Verhältnis | Pentasil-typ % | gef. $SiO_2$ % | gef. $Al_2O_3$ % | gef. $SiO_2/Al_2O_3$ % | gef. $Na^+$ % |
|---|---|---|---|---|---|---|---|
| 1 | Ethylenglykoldimethylether | 40 | 100 | 90,2 | 4,0 | 38,2 | 2,0 |
| 2 | Diethylenglykoldimethylether | 40 | 100 | 92,2 | 3,6 | 43,9 | 2,0 |
| 3 | Triethylenglykoldimethylether | 40 | 100 | 93,1 | 3,47 | 45,6 | 1,84 |
| 4 | Tetraethylenglykoldimethylether | 40 | 100 | 92,9 | 3,24 | 48,7 | 1,55 |
| 5 | Tetrahydrofuran | 40 | 100 | 92,5 | 3,94 | 39,9 | 1,57 |
| 6 | Diethylether | 40 | 100 | 89,9 | 3,78 | 40,4 | 1,82 |
| 7 | Sepasolv MPE | 40 | 100 | 92,6 | 4,0 | 39,4 | 2,15 |
| 8 | Pluriol E 200 | 40 | 100 | 89,6 | 3,85 | 39,6 | 2,0 |
| 9 | Pluriol P 600 | 40 | < 100 | 93,1 | 3,0 | 53,5 | 1,82 |
| 10 | Methylal | 40 | < 100 | 90,2 | 2,8 | 54,5 | 1,6 |

Tabelle 2

| Beispiel | Aerosil g | $Al(NO_3)_3 \cdot 9\ H_2O$ g | eingesetztes $SiO_2/Al_2O_3$ | NaOH g | Pentasil-typ % | gef. $SiO_2$ % | gef. $Al_2O_3$ % | gef. $SiO_2/Al_2O_3$ | gef. $Na^+$ % |
|---|---|---|---|---|---|---|---|---|---|
| 11 | 26,7 | 16,66 | 20 | 1,77 | 100 | 84,4 | 5,4 | 26,6 | 2,1 |
| 12 | 26,7 | 5,6 | 60 | 1,2 | 100 | 91,1 | 2,7 | 57,4 | 1,4 |
| 13 | 26,7 | 4,5 | 75 | 1,42 | 100 | 95,1 | 2,2 | 73,5 | 1,0 |

Beispiele 14-15

Die in den Beispielen 1, 2 und 5 hergestellten Zeolithe werden mit Pural SB im Verhältnis 60 : 40 mit 3 % Ameisensäure als Peptisierungsmittel zu 2 mm-Strängen verstrangt und mit 20 %iger $NH_4Cl$-Lösung kontinuierlich 2 Stunden bei 80 °C ausgetauscht. Verhältnis Zeolith zu $NH_4Cl$ beträgt 1 : 15. Die so erhaltene $NH_4$-Form des Zeolithen wird getrocknet (110 °C/16 h) und calciniert (500 °C/5 h). Diese Kontakte werden für die Gasphasenalkylierung des Benzols mit Ethylen eingesetzt unter folgenden Bedingungen : $C_6H_6$ : $C_2H_4$ = 3 : 1, WHSV = 7,8 g $C_6H_6$/g Kontakt und Stunde und T = 400 °C. Die Ergebnisse sind in Tabelle 3 wiedergegeben.

Beispiele 16-18

Die in den Beispielen 14 und 15 verwendeten Kontakte werden ebenso wie ein in THF hergestellter Zeolith (mit Pural SB im Verhältnis 60 : 40 verstrangt) für die Methanolkonvertierung zu Olefinen eingesetzt. Ein Methanol/$H_2O$-Gemisch im Verhältnis 1 : 2 wird unter isothermen Bedingungen mit einer Belastung LHSV = 1,3 $h^{-1}$ (bez. reines $CH_3OH$) bei 400 °C über die genannten Kontakte geleitet. Die in Tabelle 4 aufgezeigten Produktverteilungen in der Gasphase können erzielt werden.

(Siehe Tabellen 3, 4, Seite 7 ff.)

Tabelle 3

| Beispiel | Zeolith aus Beispiel | hergestellt in | Umsatz $C_6H_6$ % | Selektivität EB bez. $C_6H_6$ % | Selektivität EB + DEB bez. $C_6H_6$ % | Umsatz $C_2H_4$ % | Selektivität EB bez. $C_2H_4$ % | Selektivität EB+DEB bzw. $C_2H_4$ % |
|---|---|---|---|---|---|---|---|---|
| 14 | 1 | Monoglym | 27 | 95 | 99 | 95,5 | 83 | 91 |
| 15 | 2 | Diglym | 26 | 97,5 | 99 | 96 | 80 | 89 |

EB = Ethylbenzol
DEB = Diethylbenzol

Tabelle 4

| Beispiel | Zeolith hergestellt in | Umsatz $CH_3OH$ % | $CH_4$ Vol.-% | $C_2H_6$ Vol.-% | $C_2H_4$ Vol.-% | $C_3H_8$ Vol.-% | $C_3H_6$ Vol.-% | $C_4^=$ Vol.-% | $C_4^+$ Vol.-% |
|---|---|---|---|---|---|---|---|---|---|
| 16 | Monoglym | 100 | 6,7 | 0,9 | 30,7 | 12,7 | 21,9 | 7,9 | 13,8 |
| 17 | Diglym | 100 | 14,5 | < 0,5 | 44,8 | 4,3 | 19,7 | 3,5 | 6,4 |
| 18 | THF | 100 | 9,4 | 1,0 | 30,8 | 13,1 | 13,5 | 4,0 | 19,0 |

**Ansprüche**

1. Verfahren zur Herstellung eines kristallinen Aluminosilikates vom Pentasiltyp durch hydrothermale Kristallisation von $SiO_2$ und $Al(OH)_3$ in Gegenwart von Alkalisalzen bei Temperaturen von 100 bis 250 °C, dadurch gekennzeichnet, daß man die Kristallisation in etherischer oder wäßrig-etherischer Mischung ausführt.

2. Verfahren nach Anspruch 1, dadurch gekennzeichnet, daß man als Lösungsmittel für die Kristallisation einen Ether der Gruppe der Ethylenglykoldimethylether bzw. ein wäßrig-etherisches Gemisch dieser Ether verwendet.

3. Verfahren nach Anspruch 1, dadurch gekennzeichnet, daß man als Lösungsmittel für die Kristallisation Diethylenglykoldimethylether bzw. ein wäßrig-etherisches Gemisch verwendet.

4. Verfahren nach Anspruch 1 bis 3, dadurch gekennzeichnet, daß die Mischung Alkalihydroxid in Mengen von 1 bis 3 Mol pro Mol $Al_2O_3$ enthält.

5. Verfahren nach Anspruch 1 bis 4, dadurch gekennzeichnet, daß man in der Reaktionsmischung ein molares Molverhältnis von $SiO_2$ zu $Al_2O_3$ von 20 bis 200 einstellt.

6. Verwendung der nach Anspruch 1 bis 5 hergestellten kristallinen Aluminosilikate als Katalysatoren für die Alkylierung von Aromaten und die Herstellung von Olefinen und/oder Aromaten aus Methanol bzw. Dimethylether.

**Claims**

1. A process for the preparation of a crystalline aluminosilicate of the pentasil type by hydrothermal crystallization of $SiO_2$ and $Al(OH)_3$ in the presence of an alkali metal salt at from 100 to 250 °C, wherein the crystallization is carried out in a mixture with an ether or an aqueous ether.

2. A process as claimed in claim 1, wherein the solvent used for the crystallization is an ether from the group of the ethylene glycol dimethyl ethers or a mixture of these ethers with water.

3. A process as claimed in claim 1, wherein the solvent used for the crystallization is diethylene glycol dimethyl ether or a mixture of this ether with water.

4. A process as claimed in claims 1 to 3, wherein the mixture contains from 1 to 6 moles of alkali metal hydroxide per mole of $Al_2O_3$.

5. A process as claimed in claims 1 to 4, wherein a molar ratio of $SiO_2$ to $Al_2O_3$ of from 20 to 200 is employed in the reaction mixture.

6. Use of a crystalline aluminosilicate prepared as claimed in claims 1 to 5 as a catalyst for the alkylation of aromatics and for the preparation of olefins and/or aromatics from methanol and/or dimethyl ether.

**Revendications**

1. Procédé pour la préparation d'un aluminosilicate cristallin du type pentasile, par cristallisation hydrothermique de $SiO_2$ et $Al(OH)_3$ en présence de sels alcalins à une température comprise entre 100 et 250 °C, caractérisé en ce qu'on effectue la cristallisation en mélange éthérique ou éthérique-aqueux.

2. Procédé selon la revendication 1, caractérisé en ce qu'on utilise, comme solvant pour la cristallisation, un éther du groupe des éthers diméthyliques d'éthylène-glycol ou un mélange éthérique-aqueux de ces éthers.

3. Procédé selon la revendication 1, caractérisé en ce qu'on utilise, comme solvant pour la cristallisation, de l'éther diméthylique de diéthylène-glycol ou un mélange éthérique-aqueux de celui-ci.

4. Procédé selon l'une quelconque des revendications 1 à 3, caractérisé en ce que le mélange contient un hydroxyde alcalin dans des proportions de 1 à 3 mol par mol d'$Al_2O_3$.

5. Procédé selon l'une quelconque des revendications 1 à 4, caractérisé en ce qu'on règle, dans le mélange réactionnel, un rapport molaire de $SiO_2$ à $Al_2O_3$ de 20 à 200.

6. Utilisation des aluminosilicates cristallins, préparés selon l'une quelconque des revendications 1 à 5, comme catalyseurs pour l'alcoylation de carbures aromatiques et pour la préparation d'oléfines et/ou de carbures aromatiques à partir de méthanol ou d'éther diméthylique.